# EUROPEAN PATENT APPLICATION

(11) **EP 3 915 589 A1**
(43) Date of publication of application: **01.12.2021**
(21) Application number: 20177188.8
(22) Date of filing: 28.05.2020
(51) Int. Cl.: A61K 49/10, C07C 229/16, C07C 237/04

(54) **TCDTA-DERIVED FE(III) COMPLEXES FOR USE IN MAGNET RESONANCE IMAGING WITH LIVER AND KIDNEY EXCRETION**

(71) Applicant: Charité - Universitätsmedizin Berlin, 10117 Berlin (DE)
(72) Inventor: HAMM, Bernd, 12207 Berlin (DE); SCHELLENBERGER, Eyk, 10115 Berlin (DE); SCHNORR, Jörg, 16515 Oranienburg OT Lenitz (DE); HAUPTMANN, Ralf, 12203 Berlin (DE); HÄCKEL, Akvile, 06886 Lutherstadt Wittenberg (DE); XIE, Jing, 10625 Berlin (DE)
(74) Representative: Gulde & Partner

(57) **Abstract**

The present disclosure provides novel Fe(III)-complexes according to Chemical Formula (I): 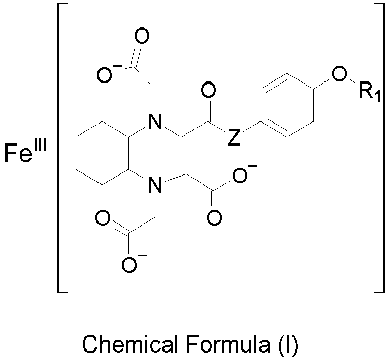 as well as enantiomers (and diastereomers) and solvates thereof. In Chemical Formula (I), Z is selected from O, NH and NR, wherein R is a branched or linear C1-C15 alkyl. R¹ is C1-C15 alkyl. Further, the orientation of the amine groups of the cyclohexyl ring in Chemical Formula (I) defines a trans-isomer. A further aspect of the disclosure refers to a contrast agent for magnet resonance imaging, which includes the Fe(III)-complex according to the present disclosure. In addition, the present disclosure provides novel ligands according to Chemical Formula (II), as well as (diastereomers and) enantiomers thereof, which constitute intermediate products for preparation of prespecified Fe(III)-complexes.

## Description

### Field of the Invention

Novel low molecular weight paramagnetic contrast agents based on novel ferric complexes for use in magnet resonance imaging (MRI) for combined imaging of the liver, the bile duct, the intestine as well as the vasculature, kidneys, the urinary system, and other organs.

### Technological Background

Low molecular weight paramagnetic contrast agents, in particular gadolinium-based contrast agents (GBCA), have been established as standard tool for improving the informative value of magnet resonance imaging (MRI) in the field of medical diagnostics. In 2015, over 10 million diagnostics were carried out by MRI in Germany. In this context, the intravenous administration of GBCA for T1-weighted imaging accounts for about one third and provides improved diagnostics for acute or chronic inflammations, tumours, neovascularization and more.

Still, non-complexed gadolinium ions are toxic, because of their chemical similarity to calcium ions. In particular, the similarity of the ion radii may result in an accumulation of gadolinium ions in the liver or the skeletal system, where the ions can remain for many years. Furthermore, gadolinium ions can act as a calcium-antagonist and thus inhibit the myocardial contractility or (blood) coagulation system.

Despite the mentioned toxicity, GBCAs have been approved for pharmaceutical use under the proviso that the toxic gadolinium ions are irreversibly complexed by means of chelating ligands such as DTPA (diethylenetriaminepentaacetic acid) or DOTA (1,4,7,10-tetraazacyclododecane-1,4,7,10-tetraacetic acid), which enable a rapid and complete excretion of the gadolinium complexes via mainly the kidneys.

However, recent studies suggest that under certain circumstances the excretion may not be complete. In fact, a number of cases of the so-called nephrogenic systemic fibrosis in patients with impaired renal function are considered a direct consequence of an incomplete excretion of some GBCAs. Further, *in vivo* testing provided experimental evidence that in skin biopsy of rats the gadolinium complexes could be detected even one year after its administration. In this context, the magnitude of gadolinium accumulation highly depends on the chelating ability of the ligands, respectively the stability of the gadolinium complexes.

Especially in cases of repeated administrations of low molecular weight contrast agents, e.g. in breast tumour screening or in follow-up controls of multiple sclerosis, accumulation of contrast agents could be observed in brain tissue of otherwise healthy patients. By means of ICP-MS (inductive coupled plasma mass spectrometry) it was also possible to detect gadolinium accumulation from stabilized macrocyclic GBCA in bone and brain samples from deceased patients.

In earlier studies it could be shown that low molecular weight ferric complexes, such as Fe(III)-DTPA and Fe(III)-tCDTA, can be used as contrast agents in MRI.

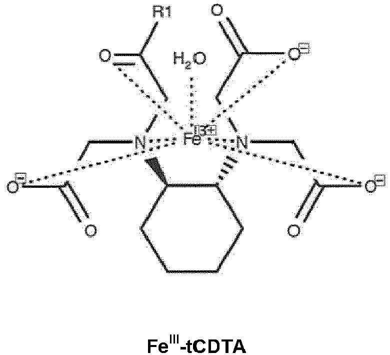

The relatively lower relaxivity of the ferric complexes compared to the gadolinium derivatives can be compensated by increased doses. It is considered that the risk of long-term toxicities of ferric complexes is lower comparted to GBCAs due to the high amount of natural iron content in human body (approx. 4 g in case of an adult) and the corresponding systems for uptake, storage and transport. Furthermore, the tolerance of iron oxide nanoparticles, which will almost completely remain in the body after intravenous administration, is generally high.

In a recent study the applicant could show that ferric complexes may also be used in modern MRI techniques such as dynamic contrast enhanced MRI (DCE-MRI) and MR angiography.

It is noteworthy that the prior art of Fe(III)-based contrast agents are mainly excreted via the kidney. That is, these complexes are not particularly suitable to examine organs such as the liver or organs of the bile duct, including the gall bladder, or the intestine. To date, the only approved signal enhancing T1 contrast agents dedicated for MRI liver diagnostics are represented by GBCAs such as Gadoxetic acid (Primovist, EOvist) and Gadobenic acid (Multihance) in Europe. However, both gadolinium complexes are less stable then macrocyclic GBCAs, which bears a higher risk of toxification.

This means there is a continuing demand for improved contrast agents in order to provide reliable and safer alternatives for (combined) MRI of the liver, the bile duct, the intestine as well as the vasculature, kidneys, the urinary system and other organs.

### Summary of Invention

The present disclosure provides novel Fe(III)-complexes according to Chemical Formula (I): 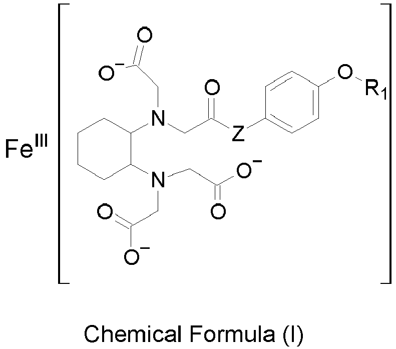 as well as enantiomers (and diastereomers) and solvates thereof. In Chemical Formula (I), Z is selected from O, NH and NR, wherein R is a branched or linear C1-C15 alkyl. R¹ is C1-C15 alkyl. Further, the orientation of the amine groups of the cyclohexyl ring in Chemical Formula (I) defines a trans-isomer.

The Fe(III)-complex may be used in in vivo diagnostic, in particular may be used in magnet resonance imaging (MRI).

A further aspect of the disclosure refers to a contrast agent for magnet resonance imaging (MRI), which includes the Fe(III)-complex according to the present disclosure. The contrast agent may be used in a method of diagnosis in vivo of acute inflammation, chronic inflammation, tumour cells and neovascularization. The contrast agent may further be used in imaging of a vascular system of mammals. In addition, the contrast agent may be used for imaging a biliary system of mammals. Furthermore, the contrast agent may be used for imaging at least one of liver, bile duct, gall bladder, pancreas, urinary bladder, and small intestine of mammals.

In addition, the present disclosure provides novel ligands according to Chemical Formula (II), as well as (diastereomers and) enantiomers thereof, which constitute intermediate products for preparation of prespecified Fe(III)-complexes.

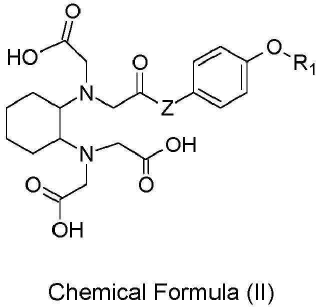

In Chemical Formula (II), Z is selected from O, NH, and NR, wherein R is a branched or linear C1-C15 alkyl. R¹ is a branched or linear C1-C15 alkyl. Further, the orientation of the amine groups of the cyclohexyl ring in Chemical Formula (II) defines a trans-isomer.

Further preferred embodiments of the present disclosure may be derived from the detailed description.

### Brief Description of the Drawings

Figure 1 shows the contrast effect of Fe(III)[4-ethoxyaniline-tCDTA] in T1-weighted MRI pictures of the liver of a mouse before the intravenous administration (A); 10 min after (B) and 22 min (C) after the intravenous administration (0.2 mmol/kg body weight).
Figure 2 shows the maximum intensity projection (MIP) of a mouse before (A) and 22 min after Fe(III)[4-ethoxyaniline-tCDTA] administration (B); intravenous administration: (0.2 mmol/kg body weight).

### Detailed Description of the Invention

With the novel Fe(III)-complexes (ferric complexes) according to Chemical Formula (I), the present invention provides remarkably stable chelate complexes due to the increased rigidity of the ligand structure and thus higher degree of pre-organization around the metal ion:

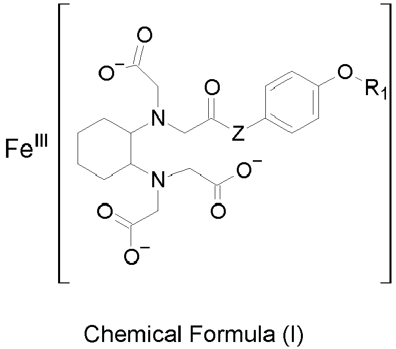

In Chemical Formula (I), Z is selected from O, NH and NR, wherein R is a branched or linear C1-C15 alkyl. R¹ is a branched or linear C1-C15 alkyl. Preferably R¹ may be a branched or linear C1-C6 alkyl. In a preferred embodiment R¹ may be ethyl. In another preferred embodiment R¹ may be ethyl, wherein Z may be selected from O or NH. In yet another embodiment R¹ may be ethyl, wherein Z may be selected from O, NH and NR with R being a branched or linear C1-C15 alkyl.

In the context of the present disclosure "C1-C15 alkyl" corresponds to a linear or branched alkyl moiety, according to CₙH₂ₙ₊₁ with n = 1 to 15. For example, C1-C6 alkyl comprises methyl, ethyl, n-propyl, 2-propyl, n-butyl, 2-butyl, 2-methylpropyl, 2-methyl-2-propyl, n-pentyl, 2-pentyl, 3-pentyl, 2-methylbutyl, 3-methylbutyl, 2-methyl2-butyl, 3-methyl-2-butyl, 2,2-dimethylpropyl, n-hexyl, 2-hexyl, 3-hexyl, 2-methylpentyl, 2-ethylbutyl, 3-methyl-2-pentyl, 2-methyl-2-pentyl, 2-methyl-3-pentyl and 4-methyl-3-heptyl. Furthermore, examples of alkyl with n ≥ 7 comprise n-heptyl, n-octyl, n-nonyl, n-decyl, 2-heptyl, 2-octyl, 2-nonyl, 2-decyl, 2-undecyl, 3-heptyl, 3-octyl, 3-nonyl, 3-decyl, 3-undecyl, 3-dodecyl, 2-methylhexl, 2-methylheptyl, 2-methyloctyl, 2-methylnonyl, 2-methyldecanyl, 2-ethylpentyl, 2-ethylhexyl, 2-ethylheptyl, 2-ethyloctyl, 2-ethylnonyl, 2-ethyldecanyl, 2-methyl-2-hexyl, 2-methyl-2-heptyl, 2-methyl-2-octyl, 2-methyl-2-nonyl, 2-methyl-2-decyl, 2-methyl-2-undecyl, 3-methyl-2-hexyl, 3-methyl-2-heptyl, 3-methyl-2-octyl, 3-methyl-2-nonyl, 3-methyl-2-decyl, 3-methyl-2-undecyl, 3-ethyl-2-pentyl, 3-ethyl-2-hexyl, 3-ethyl-2-heptyl, 3-ethyl-2-octyl, 3-ethyl-2-nonyl, 3-ethyl-2-decyl, 3-ethyl-2-undecyl, 4-methyl-3-hexyl, 4-10 methyl-3-octyl, 4-methyl-3-nonyl, 4-methyl-3-decyl, 4-methy-3-undecyl and 4-methyl-3-dodecyl.

Further, the orientation of the amine groups of the cyclohexyl ring in Chemical Formula (I) defines a *trans*-isomer, so the amine groups are oriented in opposing sides of the ring plain. In other words, the *trans*-isomerism means, that the nitrogen-bonded carbons (i.e. the carbon-stereocenters) of the cyclohexyl moiety shown in Chemical Formula (I) have S,S-configuration or R,R-configuration.

The present invention comprises solvates of the Fe(III)-complexes, which means on the one hand that one or more solvate molecule coordinates to the metal ion centre and on the other hand that the solvate molecules are included in the crystals. Preferably, the solvate is a (mono) hydrate.

The present invention also comprises enantiomers of the ferric complex, which can be derived from variation of the configuration of the nitrogen-bonded carbons (i.e. the carbon-stereocenters) of the cyclohexyl moiety shown in in Chemical Formula (I). For example, the Fe(III)-complexes (I-1a) and (I-1b), or (I-2a) and (I-2b) each constitutes a pair of enantiomers:

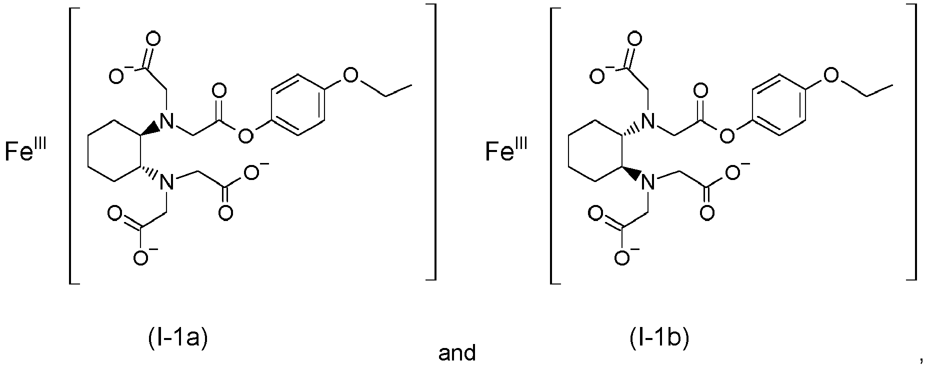

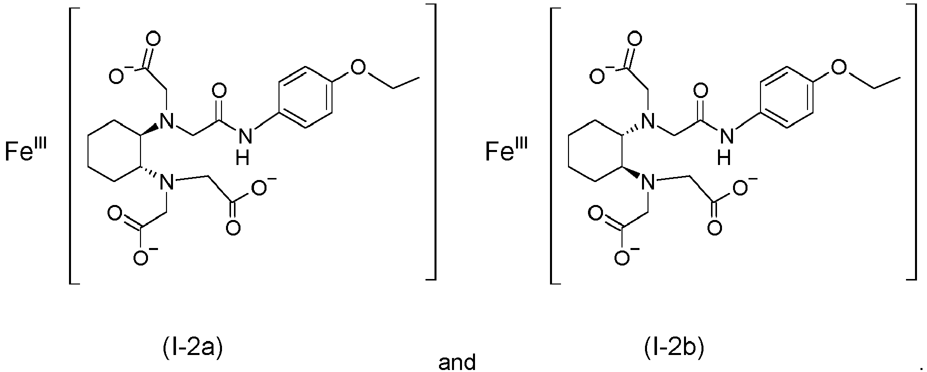

In another embodiment, the Fe(III)-complexes may be diastereomers of the ferric complex according to Chemical Formula (I). These diasteromeric ferric complexes may comprise cis-isomers of the ligand, *i.e.* with a (*R,S*) or (*S,R*) configuration of the nitrogen-bonded carbons.

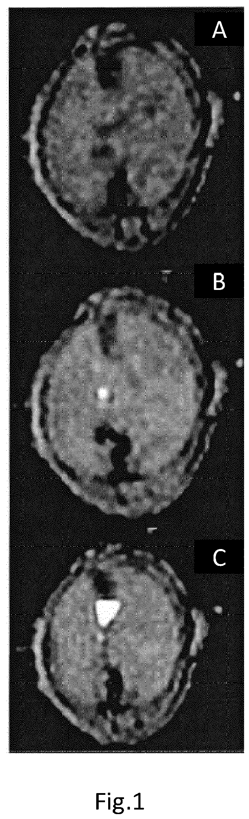

Figure 1 shows the contrast effect of Fe(III)[4-ethoxyaniline-tCDTA] in T1-weighted MRI pictures of a mouse before the intravenous administration (see Fig 1, A); 10 min after (see Fig 1, B) and 22 min (see Fig 1, C) after the intravenous administration (doses : 0.2 mmol/kg body weight) of the contrast agent. Figure 1 reveals an increasing intensity of the contrast signal in the liver of the mouse induced by the Fe(III)[4-ethoxyaniline-tCDTA] complex. The contrast signal remains high even after 22 minutes. After 22 min (see Fig 1, C), the contrast of the gallbladder is also clearly visible as round shaped signal intense region, indicating the excretion of Fe(III)[4-ethoxyaniline-tCDTA] via the liver cells.

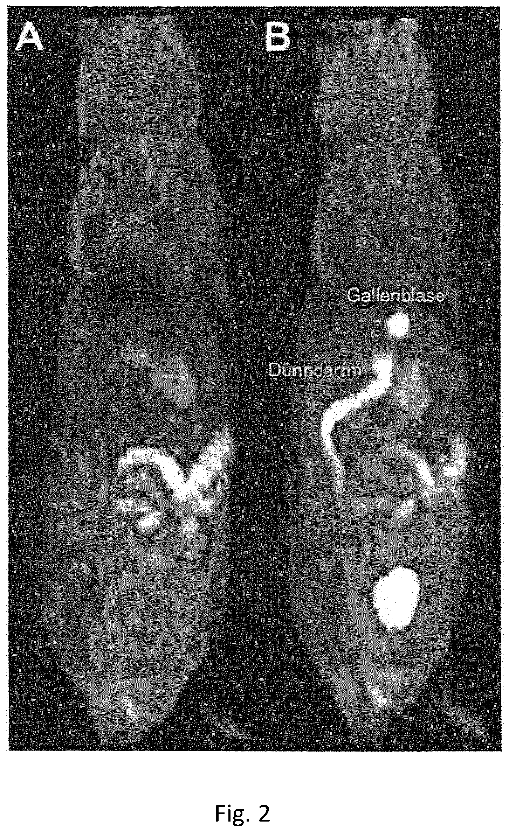

Figure 2 shows the maximum intensity projection (MIP) of a mouse before the administration of the contrast agent (see Fig.2, A) and 22 min after intravenous Fe(III)[4-ethoxyaniline-tCDTA] administration with a doses of 0.2 mmol/kg body weight (see Fig.2, B). Figure 2 reveals a strong T1-signal of the gall bladder, the urinary bladder and the small intestine after 22 min, which proves the excretion of Fe(III)[4-ethoxyaniline-tCDTA] via the liver (respectively via the liver cells) and the bile duct.

Consequently, the Fe(III)-complex according to the present invention is suitable for use in in vivo diagnostic, for example in magnet resonance imaging. In this context, the ferric complex may be used as a contrast agent or as a component of a contrast agent for magnet resonance imaging (MRI) for imaging of the vascular system, in particular of the biliary system which may include the liver, the bile duct, the gall bladder and the pancreas. Further, urinary bladder and small intestine of mammals may be investigated by MRI. For example, in case of the liver, the bile duct and the small intestine the Fe(III)-complex may be used for investigating in vivo pathological changes in these organs. Besides the specific targeting of e.g. liver cells and the resulting partial excretion by the liver, the complexes are as well suitable for MRI of other organs that are reached by the blood vessels and of the urinary system due to the partial kidney excretion. Thus, the Fe(III)-complex may be more general used as a contrast agent for imaging of the vascular system of mammals, like humans.

In particular, the Fe(III)-complex according to the present invention may be suitable for use in a method of diagnosis in vivo of acute inflammation, chronic inflammation, tumour cells and neovascularization, in particular each in the liver, the bile duct and the small intestine, the kidneys and the urinary bladder. The Fe(III)-complex according to the present invention may also be suitable for use in a method of diagnosis in vivo of acute inflammation, chronic inflammation, tumour cells and neovascularization in liver and in other organs that are reached by the blood vessels. The aforementioned method of diagnosis may be one of magnet resonance imaging (MRI), dynamic contrast enhanced MRI (DCE-MRI) and magnet resonance angiography.

The Fe(III)-complex according to the present invention may be used as a contrast agent for magnet resonance imaging of the liver, the bile duct, the urinary bladder and the small intestine. The Fe(III)-complex according to the present invention may also be used as a contrast agent for magnet resonance imaging of other organ systems that are reached by the blood vessels. In the context of the present disclosure magnet resonance imaging of the biliary system may comprise magnet resonance imaging of the liver, the bile duct, the gall bladder and the pancreas.

Preferably, the Fe(III)-complex according to the present invention may be used for magnet resonance imaging of the liver. Preferably, the Fe(III)-complex according to the present invention may be used for magnet resonance imaging of the bile duct. Furthermore, the Fe(III) complex according to the present invention may be used for magnet resonance imaging of the urinary bladder. Another use of the Fe(III) complex according to the present invention may be in magnet resonance imaging of the small intestine. The Fe(III)-complex according to the present invention may be used for magnet resonance imaging of other organ systems that are reached by the blood vessels.

The administration dose of the Fe(III)-complex according to the present invention for use as described above may be 0.02 to 1 mmol/kg bodyweight, preferably 0.1-0.3 mmol/kg bodyweight, most preferably 0.2 mmol/kg bodyweight in order to optimize the duration and intensity of the magnet resonance imaging effect.

Another aspect of the present disclosure is a contrast agent for magnet resonance imaging, comprising a Fe(III)-complex as described above. The contrast agent may further comprise a medium and the Fe(III)-complex as described above. The medium may be selected from water or solutions containing pharmaceutically acceptable salts and/or excipients. The contrast agent may be preferably administered by means of intravenous injection.

A further aspect of the present disclosure relates to a ligand according to Chemical Formula (II), which can be used as intermediate product for the synthesis of the ferric complexes according to the first aspect of this disclosure.

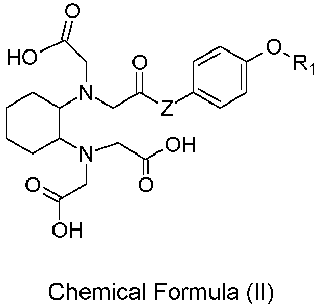

In Chemical Formula (II), Z is selected from O, NH and NR, wherein R is a branched or linear C1-C15 alkyl. R¹ is a branched or linear C1-C15 alkyl. Preferably R¹ may be a branched or linear C1-C6 alkyl. In a preferred embodiment R¹ may be ethyl. In another preferred embodiment R¹ may be ethyl, wherein Z may be selected from O or NH. In yet another embodiment R¹ may be ethyl, wherein Z may be selected from O, NH and NR with R being a branched or linear C1-C15 alkyl.

Further, the orientation of the amine groups of the cyclohexyl ring in Chemical Formula (II) defines a trans-isomer, so the amine groups are oriented to opposite sides of the ring plain. In other words, the *trans*-isomerism means, that the nitrogen-bonded carbons (i.e. the carbon-stereocenters) of the cyclohexyl moiety shown in Chemical Formula (II) have S,S-configuration or *R,R*-configuration.

The present invention also comprises enantiomers of the ligand according to Chemical Formula (II), which can be derived from variation of the configuration of the nitrogen-bonded carbons (i.e. the carbon-stereocenters) of the cyclohexyl moiety shown in in Chemical Formula (II). For example, the ligands (II-1a) and (II-1b), or (II-2a) and (II-2b) each constitutes a pair of enantiomers:

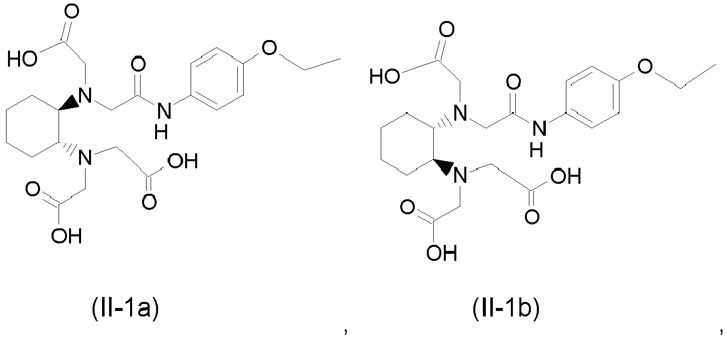

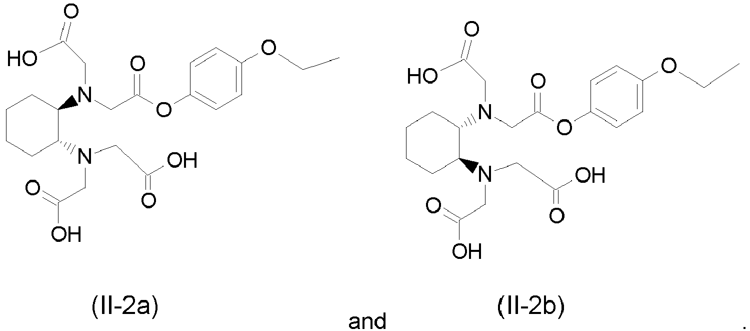

### Synthesis of 4-ethoxyanilin-trans-1,2-diaminocyclohexane-N,N',N'-triacetic acid (abbrev. 4-ethoxyaniline-tCDTA)

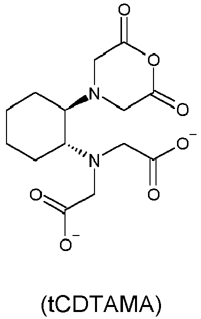

Under an argon atmosphere, 4.0 g (10.9mmol) of *trans*-1,2-diaminocyclohexane-*N,N,N',N'-*tetraacetic acid was added to a solution of 7.89 ml (83.5 mmol) acetic anhydride and 1.69 ml (20.89 mmol) pyridine and stirred for 24 h at room temperature. The reaction mixture was filtered. Then the solids were washed with acetic anhydride and subsequently dried in vacuum to yield 3.16 g (79 %) of tCDTAMA as a white solid.

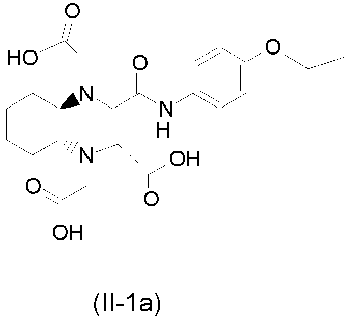

Under an argon atmosphere, 3.16 g (9.12 mmol) tCDTAMA was added portion-wise over 6 h to a solution of 23.49 mL 4-Ethoxyaniline (182 mmol). After complete addition the reaction mixture was stirred overnight at room temperature. Precipitation of the product was induced by addition of diethylether. The precipitant was filtered, washed with diethylether and dried in vacuo to yield 2.65 g (84%) of 4-ethoxyaniline-tCDTA as a racemic mixture of II-1a and II-1b as a white solid.

### Synthesis of 4-ethoxyphenol-trans-1,2-diaminocyclohexane-N,N',N'-triacetic acid (abbrev. 4-ethoxyphenol-tCDTA)

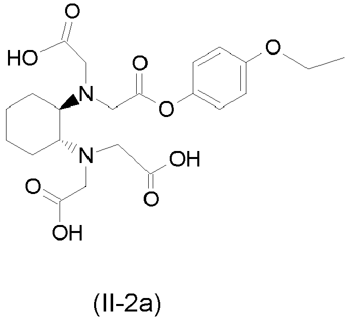

Under an argon atmosphere, 1.58 g (4.56 mmol) tCDTAMA was added portion-wise over 6 h to a prewarmed solution of 9.14 g 4-Ethoxyphenol (66.1 mmol) at 90°C.

After complete addition the reaction mixture was stirred for 24h. After cooling to RT the product was precipitated by addition of diethylether. The precipitant was filtered, washed and recrystallized in diethylether to yield 4-ethoxyaniline-tCDTA as a racemic mixture of II-2a and II-2b as a beige solid.

### Syntheses of Fe(III)complexes

Fe(III)[4-ethoxyaniline-tCDTA] or Fe(III)[4-ethoxyphenol-tCDTA] can be synthesised according to the procedure of US5362475A starting from FeCI3 as the Fe(III)source. In alternative it can be derived from Fe(III)-hydroxide according to PCT/EP2019/064750.

### Relaxivities of Fe(III)[4-ethoxyaniline-tCDTA]

**Table 1: Relaxivities (r1 and r2) of the iron chelate of 4-Ethoxyanilin-tCDTA [L • mmol⁻¹• s⁻¹] per metal ion determined at 0.94, 1.5, 3.0, and 7.0 T**

| **Field strength (temperature)** | **Relaxivity in Solvent** | **4-Ethoxyaniline-tCDTA** [L • mmol⁻¹ • s⁻¹] |
|---|---|---|
| | r1 in water | 1.43 ± 0.05 |
| **0.94 T** | r1 in serum | 1.95 ± 0.07 |
| **37°C** | r2 in water | 1.56 ± 0.01 |
| | r2 in serum | 2.09 ± 0.17 |
| **1.5 T** | r1 in water | 1.83 ± 0.10 |
| **23°C** | r1 in serum | 2.10 ± 0.12 |
| **3T** | r1 in water | 1.85 ± 0.26 / 1.86 ± 0.17 |
| **37°C / 23°C** | r1 in serum | 2.10 ± 0.23 / 2.17 ± 0.10 |
| **7T** | r1 in water | 1.77 ± 0.07 / 2.70 ± 0.28 |
| **37°C / 23°C** | r1 in serum | 2.13 ± 0.26 / 3.66 ± 0.14 |

## Claims

1. A Fe(III)-complex according to Chemical Formula (I), enantiomers and solvates thereof: 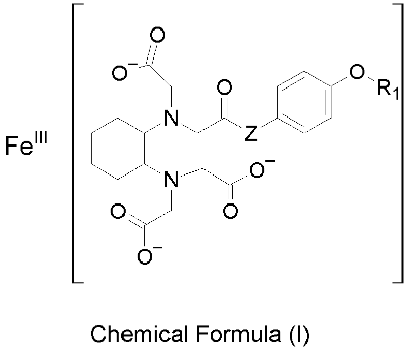 with
Z selected from O, NH and NR, wherein R is a branched or linear C1-C15 alkyl;
R¹ is a branched or linear C1-C15 alkyl; and
wherein the amine groups of the cyclohexyl ring define a trans-isomerism.

2. The Fe(III)-complex according to claim 1, wherein R¹ is a branched or linear C1-C6 alkyl.

3. The Fe(III)-complex according to claim 1, selected from the group of compounds and solvates thereof:

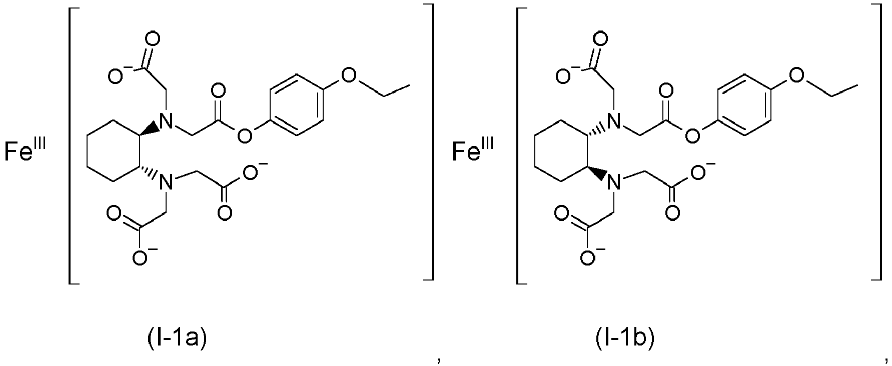

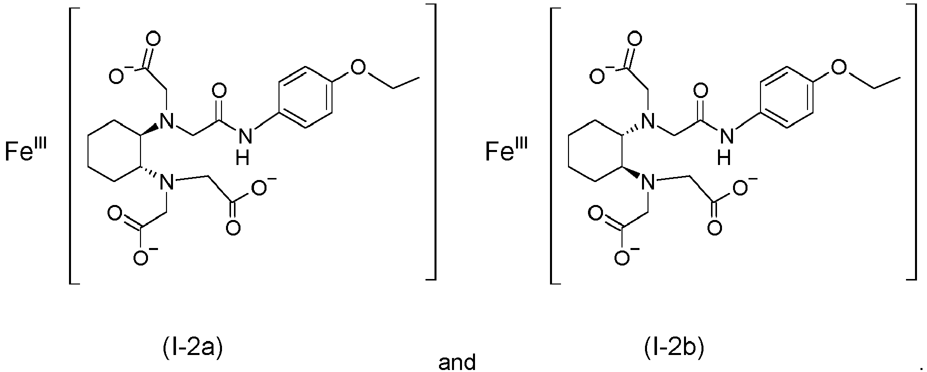

4. The Fe(III)-complex according to one of the preceding claims, wherein the solvate is a hydrate.

5. A Fe(III)-complex according to one of the preceding claims for use in in vivo diagnostic use.

6. A contrast agent for magnet resonance imaging including a Fe(III)-complex according to any one of claims 1 to 4.

7. A contrast agent according to claim 6 for use in magnet resonance imaging (MRI).

8. The contrast agent according to claim 7 for use in a method of diagnosis in vivo of acute inflammation, chronic inflammation, tumour cells and neovascularization.

9. The contrast agent according to claim 7 for use in imaging of a vascular system of mammals.

10. The contrast agent according to claim 7 for use in imaging of a biliary system of mammals.

11. The contrast agent according to claim 7 for use in imaging of at least one of liver, bile duct, gall bladder, pancreas, urinary bladder, and small intestine of mammals.

12. The contrast agent according to claim 11 for use in imaging of liver.

13. A ligand according to Chemical Formula (II), enantiomers and solvates thereof: 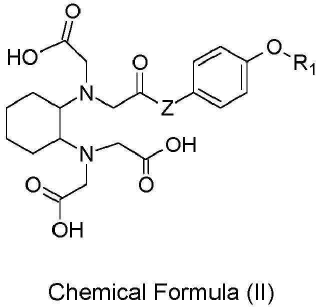 with
Z selected from O, NH and NR, wherein R is a branched or linear C1-C15 alkyl;
R¹ is a branched or linear C1-C15 alkyl; and
wherein the amine groups of the cyclohexyl ring define a trans-isomerism.

14. The ligand according to claim 13, wherein R¹ is a branched or linear C1-C6 alkyl.

15. The ligand according to claim 13, selected from the group of compounds:
